# EUROPEAN PATENT APPLICATION

(11) **EP 4 417 213 A1**
(43) Date of publication of application: **21.08.2024**
(21) Application number: 22880094.2
(22) Date of filing: 19.09.2022
(51) Int. Cl.: A61K 35/74, A61P 1/12, C12N 1/20, C12R 1/01

(54) **USE OF BACTEROIDES FRAGILIS IN PREVENTION AND TREATMENT OF CANCER-RELATED DIARRHEA**

(30) Priority: 12.10.2021 CN 202111188989
(71) Applicant: Guangzhou Zhiyi Biotechnology Co., Ltd., Guangzhou, Guangdong 510530 (CN)
(72) Inventor: LIU, Yangyang, Guangzhou, Guangdong 510530 (CN); ZHENG, Lijun, Guangzhou, Guangdong 510530 (CN); CHANG, Xiujuan, Guangzhou, Guangdong 510530 (CN); LIANG, Debao, Guangzhou, Guangdong 510530 (CN); WANG, Ye, Guangzhou, Guangdong 510530 (CN); ZHI, Fachao, Guangzhou, Guangdong 510530 (CN)
(74) Representative: Gille Hrabal Partnerschaftsgesellschaft mbB Patentanwälte
(86) International application number: PCT/CN2022/119521
(87) International publication number: WO 2023/061154

(57) **Abstract**

Provided herein is the use of Bacteroides fragilis in the prevention and treatment of cancer treatment-induced diarrhea. Specifically, provided is the use of Bacteroides fragilis in cancer treatment-induced diarrhea. In addition, further provided herein is the use of Bacteroides fragilis in the preparation of a composition for preventing and/or treating side effects of chemotherapy or targeted drugs, such as food intake change, body weight change, diarrhea, inflammatory factor increase, intestinal mucosa injury, mucosa congestion, inflammatory cell infiltration and/or gland damage.

## Description

The present application claims the benefit of priority of the prior application No. 202111188989.X entitled "USE OF BACTEROIDES FRAGILIS IN PREVENTION AND TREATMENT OF CANCER-RELATED DIARRHEA" and filed to the China National Intellectual Property Administration on October 12, 2021, which is incorporated herein by reference in its entirety.

### TECHNICAL FIELD

The present disclosure relates to use of *Bacteroides fragilis,* particularly in the prevention and treatment of cancer treatment-induced diarrhea.

### BACKGROUND

Cancer treatment-induced diarrhea (CTID) is a common toxic side effect in the digestive tract caused by chemotherapy in tumor patients. CTID not only can reduce the life quality of patients, but also can cause water and electrolyte imbalance, dehydration and infection, and can cause shock and death in severe cases. CTID is mainly secretory diarrhea (Rutledge DN, Engelking C. Cancer-related diarrhea: selected findings of a national survey of oncology nurse experiences[J]. Oncol Nurs Forum, 1998, 25(5):861-73) that usually occurs within 24-96 hours after chemotherapy or targeted drug infusion.

The pathophysiology of CTID is complex, and current understanding suggests that tumor treatment-induced mucositis predominates in the development of CTID. The tumor treatment-induced mucositis results in dysbiosis of intestinal flora, impairment of the intestinal mucosal barrier (breakdown of the intestinal mucosal layer and detachment of the intestinal epithelium), and disruption of the reabsorption function of the microvillus cells, and thereby leads to diarrhea. Drugs capable of eliciting CTID include the following types: fluoropyrimidine (e.g., 5-fluorouracil and the compound capecitabine with high activity), topoisomerase inhibitors (e.g., irinotecan or topotecan), and other types (e.g., oxaliplatin, paclitaxel, cisplatin, or cytarabine), with irinotecan (CPT-11) and 5-fluorouracil (5-FU) being the most common; additionally, similar symptoms are also present for emerging targeted small molecule drugs. For instance, epidermal growth factor receptor (EGFR) targeted inhibitors (gefitinib, afatinib, etc.) are known to cause side effects that range from rashes to mucositis. The timing of intestinal mucosa barrier impairment varies depending on the specific drug.

Symptomatic treatment is mainly adopted for CTID, with conventionally used drugs including non-specific morphine analgesics, opioid receptor inhibitor loperamide, somatostatin analog octreotide, and other antidiarrheal agents, such as montmorillonite powder. Among these, loperamide serves as a first-line drug, and octreotide is recommended as a first-line drug for patients with a CTID score of grade 3 or higher. However, significant side effects are associated with both loperamide and octreotide. The side effects of loperamide include severe constipation, abdominal pain, dizziness, rash, and the exacerbation of pre-existing conditions such as abdominal distension, nausea, and vomiting, among others. The side effects of octreotide include slow and/or uneven heartbeat, severe constipation, stomach pain, thyroid enlargement, vomiting, nausea, headache, and dizziness, among others. Meanwhile, the effectiveness of montmorillonite powder against severe diarrhea is minimal.

Symbiotic bacteria play an important role in intestinal homeostasis and have a certain protective effect on intestinal integrity. Therefore, the use of probiotics to treat CTID has emerged as a research hotspot in recent years. Probiotics, by colonizing certain parts of the human body, alter the microbial composition at those sites and constitute a category of beneficial live microorganisms for the host. Currently, the probiotic formulations commonly utilized in the treatment of CTID include Zhengchangsheng *(Bifidobacterium* triple viable capsules), Siliankang *(Bifidobacterium* tetragenus viable bacteria tablets), and their use in combination with other probiotic formulations, such as Zhengchangsheng *(Bacillus licheniformis)*

In response to the above problems, paraprobiotics have been developed. Paraprobiotics are defined as "non-viable microbial cells that, if administered orally or topically in adequate amounts, provide a benefit to the user". This definition includes morphologically incomplete microbial cells and morphologically intact microbial cells, and according to the mainstream research view, broken microbial cells can better release functional molecules and therefore have a better effect. However, for intact inactivated microbial cells, there is no unified view that can account for their superiority over living bacteria in certain applications. At present, the research focus related to paraprobiotics is the first-generation probiotics, such as *lactobacillus, bifidobacterium,* and the research direction is relatively fixed; therefore, there is a need to develop novel paraprobiotics and expand the range of indications of paraprobiotics.

### SUMMARY

In response to the above problems, the present disclosure provides use of *Bacteroides fragilis* in the preparation of a composition for preventing and treating cancer treatment-induced diarrhea. According to an embodiment of the present disclosure, the *Bacteroides fragilis* is selected from *Bacteroidesfragilis* ZY-312 with the preservation number of CGMCC No. 10685. The present disclosure further provides use of the *Bacteroides fragilis* in the preparation of a composition for preventing and/or treating side effects of a chemotherapeutic drug or a targeted drug.

According to an embodiment of the present disclosure, the chemotherapeutic drug is selected from one or more of cytotoxic chemotherapeutic drugs, taxanes, platinum-based drugs, fluorouracils, and camptothecins; and/or, the targeted drug is selected from one or more of Anti-EGFR, Anti-HER-2, Anti-BRAF, Anti-MEK, Anti-EMI,4/ALK, Anti-VEGF, Multi-targeted TKI, Anti-mTOR, Anti-CDK4/6, and Anti-PARP.

According to an embodiment of the present disclosure, the chemotherapeutic drug is selected from one or more of docetaxel, oxaliplatin, cisplatin, fluorouracil, capecitabine, and irinotecan; and/or, the targeted drug is selected from one or more of afatinib, osimertinib, neratinib, vemurafenib, dabrafenib, cobimetinib, trametinib, crizotinib, aflibercept, lapatinib, pyrotinib, imatinib, pazopanib, everolimus, sirolimus, palbociclib, ribociclib, olaparib, and rucaparib. According to an embodiment of the present disclosure, the *Bacteroides fragilis* has the selection as indicated above. The *Bacteroides fragilis* described above is inactivated, attenuated, or low-infectivity or non-infectious, and contains a bacterial protein component with a natural structure and can effectively reduce the expression of depletion molecules on T cells and thereby enhance the T cell immunity. The *Bacteroides fragilis* is one or more of: living *Bacteroides fragilis;* inactivated, genetically recombined, engineered or modified, attenuated, chemically treated, physically treated, or inactivated *Bacteroides fragilis; Bacteroides fragilis* lysate; and/or *Bacteroides fragilis* liquid culture supernatant.

Preferably, the inactivated *Bacteroidesfragilis* is a morphologically intact inactivated bacterium and/or a morphologically incomplete inactivated bacterium,
preferably a morphologically intact inactivated bacterium.

Preferably, the *Bacteroides fragilis* described above is inactivated by any one or more of the methods including dry heat, moist heat, filtration, organic solvents, chemical reagents, ultraviolet or infrared radiation, fermentation, lyophilization, genetic recombination, and genetic modification or engineering. For example, inactivated bacteria powders are obtained by lyophilization.

In the application and/or use described above, the composition may be any one of a pharmaceutical composition, food, a healthcare product, or a food additive.

The present disclosure further provides *Bacteroides fragilis* for use in the preparation of a pharmaceutical composition for preventing and treating cancer treatment-induced diarrhea, wherein the pharmaceutical composition contains *Bacteroides fragilis* ZY-312 with the preservation number of CGMCC No. 10685.

In one embodiment, the *Bacteroides fragilis* is an inactivated bacterium obtained by lyophilization.

According to an embodiment of the present disclosure, the pharmaceutical composition further comprises a pharmaceutically acceptable carrier and/or excipients.

According to an embodiment of the present disclosure, the pharmaceutical composition is formulated as a pill, a tablet, a granule, a capsule, a powder, a suspension, an oral liquid, or an enema.

Preferably, the pharmaceutical composition is administered orally or via enema.

According to an embodiment of the present disclosure, the administration cycle of the pharmaceutical composition is intermittent administration, periodic administration, continuous administration, or chronic administration.

The present disclosure further provides a healthcare product composition for preventing and treating cancer treatment-induced diarrhea, wherein the healthcare product composition contains *Bacteroidesfragilis* ZY-312 with the preservation number of CGMCC No. 10685. The present disclosure further provides a method for preventing and treating cancer treatment-induced diarrhea, which comprises administering to a patient a therapeutically effective amount of the *Bacteroides fragilis* or the composition described above,
wherein the "preventing and treating" includes prevention and/or treatment.

### Beneficial Effects of Present Disclosure:

In the present disclosure, it is unexpectedly found that the inactivated *Bacteroides fragilis,* particularly the inactivated *Bacteroides fragilis* ZY-312 with the preservation number of CGMCC No. 10685, not only retains the living bacteria's ability to adhere to intestinal epithelial cells and protect the intestinal epithelium; but also, but also, by interacting with a Toll-like receptor (TLR) and subsequent activation of NF-κB signaling pathway, it can reduce inflammatory cytokines such as TNF-α, IL-1β, IFN-γ, IL-6, among others, thereby ameliorating intestinal inflammation, and restoring the barrier function of the intestinal mucosa. Additionally, it also features better gastric acid resistance and bile salt resistance and has the advantages of high safety, easy storage, strong stability, among others.

### BRIEF DESCRIPTION OF THE DRAWINGS

FIG. 1 is an image showing the colony characteristics of *Bacteroides fragilis* ZY-312 of Example 1.
FIG. 2 is a micrograph of *Bacteroides fragilis* ZY-312 of Example 1 after gram-staining.

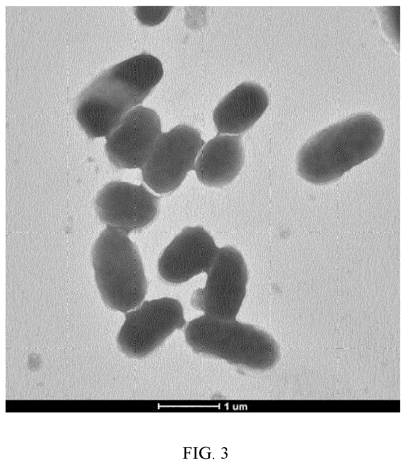

FIG. 3 is an image illustrating the morphology of the inactivated cells of *Bacteroides fragilis* of Example 2.

### DETAILED DESCRIPTION

The technical solutions of the present disclosure will be further described in detail with reference to the following specific examples. It should be understood that these following examples are provided only as illustrative and explanatory in nature for the present claimed invention and should not be interpreted as limiting the scope of the invention. All techniques realized based on the content described above fall within the intended scope of protection of the invention.

Unless otherwise stated, the starting materials and reagents used in the following examples are all commercially available products or can be prepared by using known methods.

### Example 1. Culturing of Bacteroides Fragilis by Fermentation

The *Bacteroidesfragilis* ZY-312 strain was subjected to streak inoculation on a blood agar plate and then to anaerobic culturing for 48 h. The morphological characteristics, staining characteristics, size, globular or rod-like shape, distribution, and the like of the colony were observed.

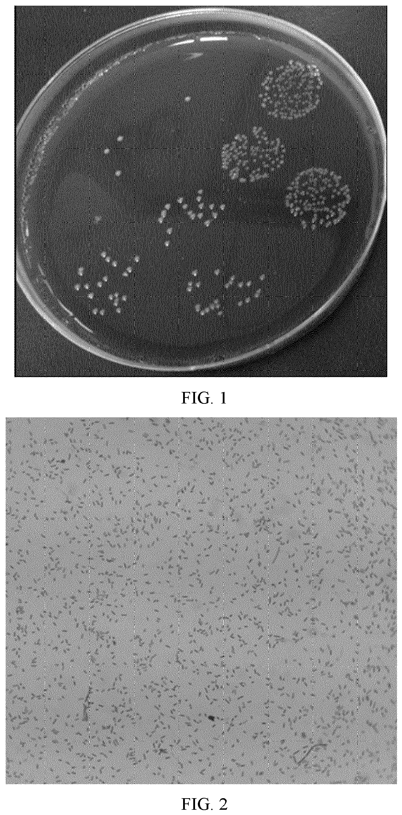

Colony characteristics: After being cultured on the blood agar plate for 48 h, the *Bacteroides fragilis* ZY-312 presented a round and slightly convex shape and was translucent, white, and nonhemolytic, and it had a smooth surface, and the diameter of the colony was 1-3 mm, as shown in FIG. 1.

Morphology under a microscope: *Bacteroides fragilis* ZY-312 was subjected to the gram-staining microscopy and was a gram-negative bacterium; it was typically rod-like, the two ends were blunt, rounded, and densely stained, and the non-staining part in the middle of the bacterium was like a bubble, as shown in FIG. 2.

A single colony was selected and inoculated in a culture medium and then subjected to culturing by fermentation for 8 h (at the temperature of 37°C), the resulting bacteria solution was centrifuged for precipitation at the rotating speed of 3000 r/min for 15 min, and the supernatant was removed and the precipitate was collected, thus obtaining the *Bacteroides fragilis* ZY-312 bacterial sludge.

### Example 2. Sample Preparation

### 1. Preparation of Bacteroides fragilis living bacteria solution, inactivated bacteria solution, and lysate solution

1) The strain was subjected to streak inoculation on a blood agar plate and then to anaerobic culturing at 37°C for 48 h.
   Colony characteristics: After 48 h of culture on a blood agar plate, the *Bacteroides fragilis ZY-*312 colonies was round, slightly convex, translucent, white, with a smooth surface and nonhemolytic. The colony measured 1-3 mm in diameter, as shown in FIG. 1.
2) Enrichment of bacteria: A single colony from the step 1) was selected and inoculated in TSB (Tryptone soy broth containing 5% fetal bovine serum) for enrichment culturing, and the resulting bacteria solution was preserved for later use.
*3) Bacteroides fragilis* living bacteria solution: The bacteria solution prepared in the step 1) was subjected to bacterial count determination by using a Mcfarland turbidimetric tube and was diluted to 10⁷ cell/mL and 10⁹ cell/mL by using normal saline and preserved for later use.
*4) Bacteroidesfragilis* inactivated bacteria solution: *the Bacteroides fragilis* inactivated bacteria solution was prepared by high temperature or ultraviolet irradiation. Specific steps: 5 mL of the *Bacteroides fragilis* living bacteria solutions at concentrations of 10⁷ cells/mL and 10⁹ cells/mL prepared in the step 2), were placed in beakers, and the beakers containing the bacteria solution were then subjected to a constant-temperature water bath at 100 °C for 20-30 min or to an ultraviolet environment for 30-60 min, thus obtaining the *Bacteroides fragilis* inactivated bacteria solution.
*5) Bacteroides fragilis* lysate solution: The *Bacteroidesfragilis* lysate solution was prepared by ultrasonication. Specific steps: 5 mL of the *Bacteroides fragilis* living bacteria solution prepared in the step 2) was lysed for 30 min using an ultrasonic instrument with an on/off cycle of 10 seconds each, and the bacteria lysis efficiency of the system reached 99%; after lysis, the lysed bacteria solution was centrifuged at 6000 rpm and 4°C for 10 min and then filtered through a 0.22 µm filter for later use.

### 2. Preparation of Bacteroides fragilis inactivated bacteria powders

(1) The *Bacteroides fragilis* fermentation liquid prepared in Example 1 was subjected to centrifugation, the wet bacteria (namely bacterial sludge) were collected, and the normal saline was added to resuspend and wash the bacterial sludge at the ratio of bacteria: normal saline = 1:(10-30) (m:v), followed by another round of centrifugation to collect the washed bacterial sludge.
(2) An excipient, a mixture of 5% maltodextrin and 0.9% sodium chloride, was added to the bacteria obtained from the step (1) at the ratio of bacteria to excipient of 1:(5-15) (m:m). After stirring and dispersion, the mixture was heat-inactivated at (70-100)±5°C for (20-40)±5 min to obtain an inactivated bacteria solution.
(3) The inactivated bacteria solution obtained from step (2) was centrifuged to collect inactivated bacterial sludge.
(4) Excipient was added to the collected inactivated bacterial sludge from step (3) to match the total weight with that of the bacteria solution before inactivation. The mixture was stirred to ensure thorough mixing, resulting in an inactivated bacteria stock solution.
(5) The inactivated bacteria stock solution obtained from step (4) was subjected to lyophilization under vacuum, namely pre-freezing at -40±2°C for 1-3 h, followed by pre-freezing at -20±2°C for 0.5-1 h, finally pre-freezing at -40±2°C for 0.5-2 h. Primary drying was conducted at -5±2°C and 0±2°Cand desorption drying at 35±2°C, all under a vacuum of 0.25 mbar, to prepare the inactivated bacteria powders, with a bacterial count of no less than 1 × 10¹¹ cells/g.

### Example 3. The Effect of Bacteroides Fragilis Bacteria Solution on Irinotecan-Induced Diarrhea in Mice

### (1) Experimental method

After the quarantine, 88 BALB/c mice were randomly divided into 8 groups according to the body weight as follows: A. normal group, B. model group, C. ZY-312 living bacteria solution (10⁷ CFU/mL) from Example 2, D. ZY-312 living bacteria solution (10⁹ CFU/mL) from Example 2, E. ZY-312 living bacteria lysate solution from Example 2, F. ZY-312 inactivated bacteria solution (10⁷ cells/mL) from Example 2, G. ZY-312 inactivated bacteria solution (10⁹ cells/mL) from Example 2, and H. positive drug Bifico group (344 mg/kg), 11 mice per group. On 1-4 d of the experiment, all mice except those in the normal group received an intraperitoneal injection of irinotecan hydrochloride solution (20 mL/kg) at a dose of 80 mg/kg once daily to establish diarrhea models. From days 1-8, the mice in each group received their respective treatments once daily according to the predetermined dose and administration route; groups A and B received oral administration of NS at a dose of 10 mL/kg as control. The diarrhea condition of the experimental mice was observed every day. On 9 d, the experiment was finished. After being fasted for no less than 12 h in advance, the mice in each group were sacrificed by cervical dislocation.

Diarrhea recording and scoring criteria: Diarrhea conditions of each experimental mouse were recorded daily, employing the diarrhea scoring methodology outlined in the study of Kurita A et al. as a reference. 0 points: normal feces or none; 1 point: mild diarrhea, with feces being slightly wet and soft; 2 points: moderate diarrhea, characterized by wet and shapeless feces with mild perianal staining; 3 points: severe diarrhea, indicated by watery feces and severe perianal staining.

**Table 1. The Effect of ZY-312 strain on diarrhea score**

| **Group** | **5 d** | **6 d** | **7 d** | **8d** |
|---|---|---|---|---|
| A. normal group | 0.0±0.0** | 0.0±0.0** | 0.0±0.0** | 0.0±0.0** |
| B. model group | 1.5±1.0 | 2.2±0.6 | 2.0±0.9 | 1.7±1.3 |
| C. ZY-312 living bacteria solution (10⁷ CFU/mL) | 0.7±1.1 | 1.741.0 | 1.5±1.5 | 1.4±1.4 |
| D. ZY-312 living bacteria solution (10⁹ CFU/mL) | 1.0±0.9 | 2.3±0.8 | 1.8±1.2 | 1.3±1.4 |
| E. ZY-312 living bacteria lysate solution | 0.5±0.5 | 1.9±1.3 | 2.0±1.1 | 1.5±1.3 |
| F. ZY-312 inactivated bacteria solution (10⁷ cells/mL) | 0.5±0.9* | 2.2±1.1 | 1.6±1.2 | 1.1±1.3 |
| G. ZY-312 inactivated bacteria solution (10⁹ cells/mL) | 1.0±0.9 | 2.0±1.2 | 1.7±1.3 | 1.2±1.2 |
| H. positive drug group | 1.7±0.6 | 2.5±0.5 | 2.1±0.7 | 1.5±1.2 |

| | | | | |
|---|---|---|---|---|
| Note: * indicates P < 0.05 and ** indicates P < 0.01, compared with the model group. | | | | |

Diarrhea scoring results shown in Table 1: Compared with the normal group, the diarrhea score of the model group was significantly increased on 5-8 d (P < 0.01). On 5 d of the experiment, the diarrhea score of the fourth sample group was significantly reduced compared with the model group (P < 0.05). On 6-8 d of the experiment, each drug group showed a reduced diarrhea score compared with the model group, but no significant difference was found between the drug groups, and the inactivated bacteria solution high dose group exhibited relatively good therapeutic effect.

The dispersing agents for the suspensions in the following examples are all normal saline.

### Example 4. Therapeutic Effect of Bacteroides Fragilis Inactivated Bacteria Powders on Mouse Models of Delayed Diarrhea Caused by Irinotecan

### (1) Experimental method

Experimental design: 100 BALB/c mice (equally divided between males and females) were selected and randomly divided into 10 groups based on sex and body weight range. These included blank group, model group, positive group 1 (Zhengchangsheng 500 mg/kg), positive group 2 (Imodium 4 mg/kg), and NCTC 9343/ZY-312 inactivated bacteria powder low dose groups (2 × 10⁵ cell/mouse), medium dose groups (2 × 10⁷ cell/mouse), and high dose groups (2 × 10⁹ cell/mouse), with 10 mice in each group (equal numbers of males and females). NCTC 9343 is a type strain derived from the American population. Except for the blank group, animals in the remaining groups were intraperitoneally injected with irinotecan at a dose of 70 mg/kg (0.14 mL/10 g) once daily for 5 consecutive days to induce a diarrhea model. Starting from 3 days before model induction, animals in corresponding groups were given oral gavage (0.1 mL/10 g of body weight) with Zhengchangsheng suspension, Imodium suspension, or NCTC 9343/ZY-312 bacteria powder suspensions at various doses (high, medium, and low doses), administered once in the morning and once in the afternoon every day for 11 consecutive days. The blank and model groups received an equivalent volume of normal saline. Animals were euthanized after the final administration. Post-administration, daily observations and recordings included general appearance, behaviors and activity, respiration, glandular secretions, and fecal conditions, with a particular focus on each animal's diarrhea status, which was recorded and scored. Following the conclusion of the modeling and the experiment, fecal samples from each animal were collected for weight and moisture content analysis. Blood samples (~0.5 mL) were collected from the orbital venous plexus under isoflurane anesthesia from all surviving animals, centrifuged to separate serum, and analyzed for cytokines such as IL-1β, IL-4, IL-5, IL-6, IL-10, IL-13, IL-17, TNF-α, INF-γ, among others, using a liquid chip kit, and ICAM-1 levels were measured using an ELISA kit. Following euthanasia, the entire intestinal tract was harvested, fixed in 10% formaldehyde solution, and subjected to HE staining for pathological examination.

### Detection method and frequency:

Food intake: Feed addition and leftovers were quantified using an electronic scale. The quantity added was recorded before the administration (D0) and again on D4. The leftovers were weighed once on D4, D8, and D11 (end of the experiment ) and these measurements were documented. The daily average food intake (g) was calculated using the formula: daily average food intake (g) = (added amount - remaining amount)/number of animals/number of days.

Diarrhea recording and scoring criteria: Diarrhea conditions in each experimental mouse were recorded daily, employing the diarrhea scoring methodology outlined in the study of Kurita A et al. as a reference. 0 points: normal feces or none; 1 point: mild diarrhea, with feces being slightly wet and soft; 2 points, moderate diarrhea, characterized by wet and shapeless feces with mild perianal staining; 3 points, severe diarrhea, indicated by watery feces and severe perianal staining.

Histopathological examination of intestinal tracts: Following euthanasia, the entire intestinal tract from all surviving animals was harvested and fixed in 10% formalin solution. Pathological tissue sections were prepared, followed by Hematoxylin and Eosin (H&E) staining. The histopathological examination of intestinal tracts was carried out under a microscope.

Data statistics and analysis: The data for body weight, food intake, diarrhea scores, pathological examination results, among others, were expressed as mean ± standard deviation (Mean ± SD) and were statistically analyzed using SPSS statistical software 25.0.

### (2) Experimental results

1) **Food intake:** the food intake of animals in each group is shown in Table 2.

**Table 2. Statistical results of food intake of animals in each group (mean ± standard deviation, n = 10)**

| Group | Daily average food intake/mouse (g) | | |
|---|---|---|---|
| | Day 4 | Day 8 | Day 11 |
| Blank group | 3.79 ± 0.21 | 3.73 ± 0.42** | 4.05 ± 0.28** |
| Model group | 3.58 ± 0.46 | 1.95 ± 0.12 | 0.52 ± 0.43 |
| Positive group 1 | 4.11 ± 0.56 | 1.77 ± 0.22 | 0.60 ± 0.41 |
| Positive group 2 | 3.84 ± 0.50 | 1.62 ± 0.17 | 0.30 ± 0.32* |
| NCTC 9343 inactivated bacteria powder low dose group | 3.92 ± 0.42 | 1.59 ± 0.23 | 0.52 ± 0.35 |
| NCTC 9343 inactivated bacteria powder medium dose group | 4.05 ± 0.57 | 1.64 ± 0.22 | 0.54 ± 0.45 |
| NCTC 9343 inactivated bacteria powder high dose group | 3.98 ± 0.36 | 1.61 ± 0.19 | 0.57 ± 0.55 |
| ZY-312 inactivated bacteria powder low dose group | 3.80 ± 0.42 | 1.79 ± 0.14 | 0.72 ± 0.23 |
| ZY-312 inactivated bacteria powder medium dose group | 4.04 ± 0.39 | 1.95 ± 0.18 | 0.86 ± 0.19 |
| ZY-312 inactivated bacteria powder high dose group | 3.82 ± 0.21 | 1.93 ± 0.21 | 0.91 ± 1.08 |

| | | | |
|---|---|---|---|
| Note: * indicates significant difference p < 0.05 and ** indicates extremely significant difference p < 0.01, compared with the model group. | | | |

On D4 (i.e., before model induction), the daily average food intake of animals in the blank group, the model group, and each treatment group didn't show significant difference (p > 0.05). Compared with the blank group, the daily food intake of the male and female animals in the model group was remarkably reduced (p < 0.01) on D8 and D11 (i.e., after model induction). On D8, there was no significant difference in food intake between each drug treatment group and the model group. On D11, the food intake of positive control group 2 was significantly lower than that of the model group; the food intake in the positive group 1 and each NCTC 9343 group was similar to that of the model group, and the food intake of each dose group of ZY-312 was higher than that of the model group.
2) **Wet weight and moisture content of feces and diarrhea score:**
The wet weight and moisture content of the feces of animals in each group are shown in Table 3.

**Table 3. Statistical results of wet weight and moisture content of feces of animals in each group (mean ± standard deviation, n = 10)**

| **Group** | **Wet weight (g)** | | **Moisture content (%)** | |
|---|---|---|---|---|
| | End of model induction | End of experiment | End of model induction | End of experiment |
| Blank group | 0.9±0.2 | 1.2±0.2** | 28.5±8.7 | 28.6±2.0* |
| Model group | 1.0±0.2 | 0.6±0.3 | 39.9±8.1 | 50.0±14.1 |
| Positive group 1 | 0.4±0.1 | 0.5±0.1 | 36.7±3.8 | 46.7±8.2 |
| Positive group 2 | 0.2±0.1 | 0.4±0.2 | 41.7±8.3 | 46.7±0.0 |
| NCTC 9343 inactivated bacteria powder low dose group | 0.3±0.1 | 0.4±0.1 | 46.2±8.2 | 44.32±7.9 |
| NCTC 9343 inactivated bacteria powder medium dose group | 0.3±0.2 | 0.3±0.1 | 44.33±10.9 | 45.47±5.3 |
| NCTC 9343 inactivated bacteria powder high dose group | 0.4±0.1 | 0.4±0.2 | 41.46±6.2 | 42.69±6.4 |
| ZY-312 inactivated bacteria powder low dose group | 0.3±0.2 | 0.4±0.2 | 42.5±5.0 | 31.7±9.6* |
| ZY-312 inactivated bacteria powder medium dose group | 0.5±0.1 | 0.5±0.2 | 43.2±8.4 | 37.5±5.0* |
| ZY-312 inactivated bacteria powder high dose group | 0.4±0.2 | 0.6±0.2 | 44.4±6.6 | 30.8±6.9* |

| | | | | |
|---|---|---|---|---|
| Note: * indicates significant difference p < 0.05 and ** indicates extremely significant difference p < 0.01, compared with the model group. | | | | |

For the wet weight of feces and the moisture content of feces, the blank group didn't show significant differences. After the end of model induction, the wet weight of the feces of each model-inducing group showed no significant difference, but the moisture content of the feces of the model group was significantly increased relative to that of the blank group, and the moisture content of the feces of each treatment group was in a descending trend relative to that of the model group, wherein the *Bacteroides fragilis* groups effectively restrained the trend of the increase of the moisture content of the feces, but the ZY-312 groups showed better effect than the NCTC 9343 groups.

**The diarrhea scores of animals in each group are shown in Table 4.**

**Table 4. Statistical results of diarrhea scores of animals in each group (mean ± standard deviation n = 5)**

| | Diarrhea determination time (day) | | | | |
|---|---|---|---|---|---|
| | 7 | 8 | 9 | 10 | 11 |
| Male | | | | | |
| Blank group | 0.0 ± 0.0** | 0.0 ± 0.0** | 0.0 ± 0.0** | 0.0 ± 0.0** | 0.0 ± 0.0** |
| Model group | 1.7 ± 0.5 | 2.0 ± 0.0 | 2.0 ± 0.0 | 2.4 ± 0.5 | 2.3 ± 0.8 |
| Positive group 1 | 0.8 ± 1.0* | 1.1 ± 1.1* | 1.4 ± 0.5* | 1.9 ± 0.3* | 2.0 ± 0.8 |
| Positive group 2 | 0.8 ± 1.0* | 0.8 ± 1.0** | 1.5 ± 0.5* | 1.9 ± 0.6 | 1.9 ± 0.5 |
| NCTC 9343 inactivated bacteria powder low dose group | 0.8 ± 0.4* | 1.2 ± 0.9* | 1.5 ± 0.5* | 2.0 ±0.7 | 2.4 ± 0.4 |
| NCTC 9343 inactivated bacteria powder medium dose group | 0.9 ± 1.1* | 1.3 ± 0.5* | 1.5 ± 0.8* | 2.0 ± 0.8 | 2.3 ± 0.8 |
| NCTC 9343 inactivated bacteria powder high dose group | 0.9 ± 0.8* | 1.2 ± 0.4* | 1.3 ± 0.7* | 1.9 ± 0.4* | 2.0 ± 0.6 |
| ZY-312 inactivated bacteria powder low dose group | 1.2 ± 1.0 | 1.0 ± 1.0* | 1.6 ± 0.5* | 1.8 ± 0.4* | 1.6 ± 0.5* |
| ZY-312 inactivated bacteria powder medium dose group | 0.0 ± 0.0* | 0.8 ± 1.0** | 1.6 ± 0.5* | 1.6 ± 0.5** | 1.5 ± 0.7* |
| ZY-312 inactivated bacteria powder high dose group | 0.4 ± 0.8** | 0.8 ± 1.0** | 1.4 ± 0.7* | 1.2 ± 0.6** | 1.4 ± 0.5* |
| Female | | | | | |
| Blank group | 0.0 ± 0.0** | 0.0 ± 0.0** | 0.0 ± 0.0** | 0.0 ± 0.0** | 0.0 ± 0.0** |
| Model group | 1.9 ± 0.3 | 2.0 ± 0.0 | 2.0 ± 0.0 | 2.3 ± 0.5 | 2.5 ± 0.5 |
| Positive group 1 | 0.4 ± 0.8** | 1.0 ± 1.1* | 1.6 ± 0.5* | 1.7 ± 0.5* | 2.0 ± 0.7 |
| Positive group 2 | 0.8 ± 1.0** | 0.6 ± 1.0** | 1.7 ± 0.5 | 2.1±0.3 | 2.1±0.5 |
| NCTC 9343 inactivated bacteria powder low dose group | 0.8 ± 0.8** | 1.2 ± 1.0* | 1.7 ± 0.8 | 1.8 ± 0.7 | 2.2 ± 0.8 |
| NCTC 9343 inactivated bacteria powder medium dose group | 0.7 ± 0.4** | 1.1 ± 0.6* | 1.6 ± 0.3* | 1.9 ± 0.6 | 2.1 ± 0.2 |
| NCTC 9343 inactivated bacteria powder high dose group | 0.6 ± 0.5** | 1.1±0.5* | 1.7 ± 0.5 | 1.7 ± 0.7* | 2.0 ± 0.8 |
| ZY-312 inactivated bacteria powder low dose group | 0.8 ± 1.0** | 1.0 ± 0.6** | 1.4 ± 0.5** | 1.7 ± 0.4* | 1.7 ± 1.1 |
| ZY-312 inactivated bacteria powder medium dose group | 0.7 ± 0.9** | 1.0 ± 1.1* | 1.2 ± 0.5** | 1.6 ± 0.4* | 1.8 ± 0.8 |
| ZY-312 inactivated bacteria powder high dose group | 0.4 ± 0.8** | 1.0 ± 1.1** | 1.3 ± 0.5** | 1.4 ± 0.5** | 1.6 ± 0.6* |

| | | | | | |
|---|---|---|---|---|---|
| Note: * indicates significant difference p < 0.05 and ** indicates extremely significant difference p < 0.01, compared with the model group. | | | | | |

The animals in the model group and each treatment group began to have diarrhea on the fourth day (D7). Compared with the blank group, the diarrhea scores of these groups were significantly increased throughout the experiment, and escalated over time, indicating that the model induction was successful.

For male animals, the diarrhea scores of the positive group 1, the positive group 2, and each dose group of NCTC 9343 were also showed an upward trend, but were all lower than that of the model group. ZY-312 groups demonstrated the ability to maintain stable diarrhea scores, showing a superior ability to mitigate the increase in diarrhea scores ,with all scores being lower than those of the NCTC 9343 groups.

For female animals, the effect of the groups described above was substantially similar, and the effect of ZY-312 groups was better than that of the positive drug groups and NCTC 9343 groups.

### 3) Inflammatory cytokines

Inflammatory cytokines for animals in each group are shown in Table 5.

**Table 5. Statistical results of inflammatory cytokines for animals in each group (mean ± standard deviation)**

| Group | n | TNF-α | IL-1β | IL-4 | IL-5 | IL-6 |
|---|---|---|---|---|---|---|
| Blank group | 11 | 0.36±0.06** | 21.35±1.36* | 6.38±0.92 | 1.70±1.22** | 2.08±0.41** |
| Model group | 14 | 0.86±0.26 | 23.37±1.15 | 6.90±1.02 | 4.31±2.54 | 26.84±23.21 |
| Positive group 1 | 12 | 0.96±0.46 | 22.64±1.25 | 6.94±1.25 | 3.45±3.18 | 54.60±68.95 |
| Positive group 2 | 9 | 1.47±1.19 | 23.07±1.70 | 7.77±1.30 | 3.20±3.51 | 24.50±25.90 |
| NCTC 9343 inactivated bacteria powder low dose group | 10 | 0.82±0.47 | 20.79±1.82 | 7.32±1.45 | 3.18±2.85 | 25.23±13.27 |
| NCTC 9343 inactivated bacteria powder medium dose group | 10 | 0.84±0.78 | 19.37±2.23 | 7.14±0.86 | 2.97±1.83 | 23.26±19.30 |
| NCTC 9343 inactivated bacteria powder high dose group | 10 | 0.87±0.42 | 21.72±1.46 | 7.28±0.97 | 3.05±1.92 | 22.49±10.66 |
| ZY-312 inactivated bacteria powder low dose group | 11 | 0.79±1.09 | 15.22±1.07 | 7.06±1.13 | 1.67±0.54** | 18.27±14.38* |
| ZY-312 inactivated bacteria powder medium dose group | 12 | 0.70±0.49 | 17.63±1.70 | 7.01±1.29 | 2.20±3.51 | 16.15±13.34* |
| ZY-312 inactivated bacteria powder high dose group | 12 | 0.75±0.40 | 15.07±3.86 | 6.86±0.95 | 2.57±2.35 | 14.55±18.16* |

continued

| Group | n | IL-10 | IL-13 | IL-17 | INF-γ | ICAM-1 |
|---|---|---|---|---|---|---|
| Blank group | 11 | 1.08±0.20* | 4.76±0.66* | 2.57±0.75 | 1.88±0.18** | 1726±837* |
| Model group | 14 | 4.20±4.02 | 11.62±6.04 | 2.65±0.33 | 2.33±0.37 | 2759±1044 |
| Positive group 1 | 12 | 10.61±13.46 | 9.27±2.70 | 2.66±0.37 | 2.20±0.33 | 2931±894 |
| Positive group 2 | 9 | 21.16±17.21 * | 6.94±1.45 | 2.65±0.66 | 2.37±0.23 | 3225±859 |
| NCTC 9343 inactivated bacteria powder low dose group | 10 | 15.67±10.32 | 9.92±4.39 | 1.98±0.23 | 2.96±0.42 | 2542±878 |
| NCTC 9343 inactivated bacteria powder medium dose group | 10 | 16.93±12.33 | 8.73±3.27 | 2.02±0.35 | 2.77±0.23 | 2577±1022 |
| NCTC 9343 inactivated bacteria powder high dose group | 10 | 18.45±9.82 | 9.64±3.96 | 1.72±0.47 | 2.84±0.47 | 2423±987 |
| ZY-312 inactivated bacteria powder low dose group | 11 | 26.74±17.69 | 12.88±3.93 | 1.50±0.20 | 2.32±0.37 | 2042±792 |
| ZY-312 inactivated bacteria powder medium dose group | 12 | 24.33±11.28 | 14.48±2.37 | 1.50±0.25 | 2.20±0.36 | 2170±795 |
| ZY-312 inactivated bacteria powder high dose group | 12 | 21.16±17.21 | 11.62±6.04 | 1.42±0.18 | 2.16±0.55 | 2022±562 |

| | | | | | | |
|---|---|---|---|---|---|---|
| Note: n is the number of animals; * indicates significant difference p < 0.05 and ** indicates extremely significant difference p < 0.01, compared with the model group. | | | | | | |

Compared with the blank group, the inflammatory cytokines (TNF-α, IL-1β, IL-5, IL-6, IL-17 and INF-γ) and ICAM-1 protein in the model group were significantly increased; the anti-inflammatory cytokines IL-4, IL-10, and IL-13 were significantly reduced. The positive drug groups had the effect of down-regulating the level of inflammatory cytokines and up-regulating the level of the anti-inflammatory cytokines. The *Bacteroides fragilis* groups also showed the effect of regulating cytokines, wherein ZY-312 exhibited a better regulatory effect than NCTC 9343. There was no significant dose difference within the groups of each *Bacteroides fragilis* strain.

4) Pathological examination: Jejunum, ileum, and colorectum tissues were selected and HE staining was performed, followed by pathological evaluation. The results are shown in Table 6.

**Table 6. Pathological examination results of animals in each group (mean ± standard deviation)**

| Group | **Jejunum** | | | | | |
|---|---|---|---|---|---|---|
| | n | Gland dilation | Intestinal mucosal damage | Inflammatory cell infiltration | Mucosal hyperemia | Mucosal hemorrhage |
| Blank group | 20 | 0±0** | 0±0** | 0.2±0.41 | 0±0* | 0±0** |
| Model group | 18 | 1.00±0.69 | 0.44±0.51 | 1.89±0.68 | 0.39±0.7 | 0.06±0.25 |
| Positive group 1 | 16 | 0.88±0.62 | 0.56±0.81 | 2.06±0.85 | 0.38±0.62 | 0±0** |
| Positive group 2 | 14 | 1.07±0.62 | 0.71±0.91 | 2.43±0.65 | 0.36±0.63 | 0±0** |
| NCTC 9343 inactivated bacteria powder low dose group | 10 | 0.72±0.62 | 0.32±0.42 | 1.75±0.72 | 0.35±0.57 | 0±0** |
| NCTC 9343 inactivated bacteria powder medium dose group | 10 | 0.77±0.37 | 0.37±0.53 | 1.63±0.83 | 0.32±0.35 | 0±0** |
| NCTC 9343 inactivated bacteria powder high dose group | 10 | 0.75±0.54 | 0.33±0.45 | 1.69±0.48 | 0.37±0.44 | 0±0** |
| ZY-312 inactivated bacteria powder low dose group | 16 | 0.65±0.58 | 0.25±0.58 | 1.46±1.03 | 0.24±0.63 | 0±0** |
| ZY-312 inactivated bacteria powder medium dose group | 20 | 0.60±0.72 | 0.25±0.64 | 1.45±0.83 | 0.20±0.47 | 0±0** |
| ZY-312 inactivated bacteria powder high dose group | 17 | 0.64±0.66 | 0.12±0.33 | 1.18±1.01* | 0.29±0.47 | 0±0** |

| Group | **Ileum** | | | | | |
|---|---|---|---|---|---|---|
| | n | Gland dilation | Intestinal mucosal damage | Inflammatory cell infiltration | Mucosal hyperemia | Mucosal hemorrhage |
| Blank group | 20 | 0±0** | 0±0** | 0.1±0.31 | 0.05±0.22* | 0±0** |
| Model group | 18 | 1.22±0.81 | 2.33±1.46 | 1.61±1.12 | 0.72±1.07 | 0.06±0.24 |
| Positive group 1 | 16 | 1.00±0.52 | 2.14±1.36 | 1.05±1.13 | 0.60±0.72 | 0±0** |
| Positive group 2 | 14 | 1.04±0.95 | 2.04±1.39 | 1.43±1.09 | 0.5±1.03 | 0±0** |
| NCTC 9343 inactivated bacteria powder low dose group | 10 | 0.93±0.47 | 1.92±0.86 | 1.52±0.97 | 0.54±0.72 | 0.02±0.22 |
| NCTC 9343 inactivated bacteria powder medium dose group | 10 | 0.92±0.85 | 1.86±0.92 | 1.61±1.02 | 0.59±0.61 | 0.03±0.58 |
| NCTC 9343 inactivated bacteria powder high dose group | 10 | 0.84±0.72 | 2.01±1.21 | 1.63±0.78 | 0.51±0.34 | 0±0** |
| ZY-312 inactivated bacteria powder low dose group | 16 | 0.86±0.74 | 1.53±1.46 | 1.36±1.09 | 0.39±0.54 | 0±0** |
| ZY-312 inactivated bacteria powder medium dose group | 20 | 0.75±0.83 | 1.35±1.53 | 1.32±0.85 | 0.35±0.38 | 0±0** |
| ZY-312 inactivated bacteria powder high dose group | 17 | 0.72±0.49 | 1.47±1.33 | 1.41±0.78 | 0.33±0.42 | 0±0** |

| Group | **Colorectum** | | | | | |
|---|---|---|---|---|---|---|
| | n | Gland dilation | Intestinal mucosal damage | Inflammatory cell infiltration | Mucosal hyperemia | Mucosal hemorrhage |
| Blank group | 20 | 0±0** | 0±0 | 0±0** | 0±0** | 0±0** |
| Model group | 18 | 1.56±1.1 | 0±0 | 0.11±0.32 | 0.17±0.38 | 0.22±0.55 |
| Positive group 1 | 16 | 1.53±1.7 | 0±0 | 0.09±0.4 | 0.05±0.58 | 0±0** |
| Positive group 2 | 14 | 1.48±1.4 | 0±0 | 0.07±0.27 | 0.05±0.85 | 0±0** |
| NCTC 9343 inactivated bacteria powder low dose group | 10 | 1.52±0.9 | 0±0 | 0.1±0.37 | 0.11±0.44 | 0.21±0.80 |
| NCTC 9343 inactivated bacteria powder medium dose group | 10 | 1.56±1.0 | 0±0 | 0.10±0.22 | 0.10±0.57 | 0±0** |
| NCTC 9343 inactivated bacteria powder high dose group | 10 | 1.61±1.1 | 0±0 | 0.09±0.23 | 0.15±0.32 | 0±0** |
| ZY-312 inactivated bacteria powder low dose group | 16 | 1.44±1.3 | 0±0 | 0.10±0.34 | 0.06±0.25 | 0±0** |
| ZY-312 inactivated bacteria powder medium dose group | 20 | 1.45±1.3 | 0±0 | 0.05±0.22 | 0.07±0.38 | 0±0** |
| ZY-312 inactivated bacteria powder high dose group | 17 | 1.36±1.1 | 0±0 | 0.06±0.24 | 0.09±0.33 | 0±0** |

| | | | | | | |
|---|---|---|---|---|---|---|
| Note: n is the number of animals; * indicates significant difference p < 0.05 and ** indicates extremely significant difference p < 0.01, compared with the model group. | | | | | | |

For the jejunum section, compared with the blank group, only the model group showed mucosal hemorrhage , although it was not pronounced, and the model showed significant pathological damages such as gland dilation, intestinal mucosal damage, inflammatory cell infiltration, mucosal hyperemia, and the like. The NCTC 9343 bacteria powder groups demonstrated certain therapeutic effect on gland dilation, intestinal mucosal damage, and inflammatory cell infiltration, and the ZY-312 groups showed therapeutic effects on the pathological damages including gland dilation, intestinal mucosal damage, inflammatory cell infiltration, and mucosal hyperemia.

For the ileum section, there was noticeable mucosal hemorrhage in the model group, not observed in the *Bacteroides fragilis* groups. The positive drug groups showed significant therapeutic effect on the remaining aspects of gland dilation, intestinal mucosal damage, inflammatory cell infiltration, and mucosal hyperemia. Both the NCTC 9343 groups and ZY-312 groups showed therapeutic effects, with the ZY-312 groups showing superior efficacy compared to NCTC 9343 groups.

For the colorectum section, no intestinal mucosal damage was found in each group. In addition, the model group exhibited gland dilation, inflammatory cell infiltration, as well as some degree of mucosal hyperemia and hemorrhage. The NCTC 9343 groups showed the effect of suppressing the deterioration of the condition, and ZY-312 also showed therapeutic effect and was better than the NCTC 9343 groups with regard to the degree of amelioration. There was no significant dose difference within the groups of each *Bacteroides fragilis* strain.

In conclusion, the *Bacteroides fragilis* inactivated bacteria powder (ZY-312/NCTC 9343) is effective in treating the cancer treatment-induced diarrhea of mice caused by irinotecan, with ZY-312 showing superior efficacy compared to NCTC 9343.

### Example 5. Therapeutic Effect of Bacteroides Fragilis Inactivated Bacteria Powder on Mouse Models of Cancer Treatment-Induced Diarrhea Caused by 5-Fluorouracil (5-FU)

### (1) Experimental method

Experimental design: In the experiment, 100 BALB/c mice (equally divided between males and female) were selected and randomly divided into 10 groups based on the sex and body weight range. These included blank group, model group, positive group 1 (Zhengchangsheng 500 mg/kg), positive group 2 (Imodium 4 mg/kg), and NCTC 9343/ZY-312 inactivated bacteria powder low dose groups (2×10⁵ bacteria/mouse), medium dose groups (2×10⁷ bacteria/mouse), and high dose groups (2×10⁹ bacteria/mouse), with 10 mice (equal numbers of males and females) in each group. Except for the blank group, animals in the remaining groups were intraperitoneally injected with 5-FU at a dose of 45 mg/kg (0.1 mL/10g) once daily for 5 consecutive days to induce a diarrhea model. Starting from 3 days before model induction, animals in corresponding groups were given oral gavage (0.1 mL/10 g of body weight) with Zhengchangsheng suspension, Imodium suspension, and NCTC 9343/ZY-312 bacteria powder suspensions at various doses (high, medium, and low doses), administered once in the morning and once in the afternoon every day for 11 consecutive days. The blank and model groups received an equivalent volume of normal saline (0.1 mL/10 g of body weight). The animals were euthanized the day after the final administration. Daily observation and recording included general appearance, behaviors and activity, respiration, glandular secretion, fecal condition, with a particular focus on each animal's diarrhea status, which was recorded and scored. Following the conclusion of the modeling and the experiment, fecal samples from each animal were collected for weight and moisture content analysis. Blood samples (~0.5 mL) were collected from the orbital venous plexus under isoflurane anesthesia from all surviving animals, centrifuged to separate serum, and analyzed for cytokines such as IL-1β, IL-4, IL-5, IL-6, IL-10, IL-13, IL-17, TNF-α, and INF-γ using a liquid chip kit and ICAM-1 levels were measured using an ELISA kit. Following euthanasia, the entire intestinal tract was harvested, fixed in 10% formaldehyde solution, and subjected to HE staining for pathological examination.

### Detection method and frequency:

Determination of moisture content of feces: Feces were collected over a period of 4 h from each mouse housed in separate cages in the afternoon of D8 (end of model induction) and D11, then the wet weight was measured. Subsequently, the feces were dried in an oven at 80 °C for 3 h, after which the dry weight was measured and recorded. The calculation was carried out according to the following formula: moisture content of feces (%) - (wet weight of feces - dry weight of feces)/wet weight of feces × 100.

Diarrhea recording and scoring criteria: Diarrhea conditions in each experimental mouse was recorded daily, employing the diarrhea scoring methodology outlined in the study of Kurita A et al. as a reference. 0 points: normal feces or none; 1 point: mild diarrhea, with feces being slightly wet and soft; 2 points: moderate diarrhea, characterized by wet and shapeless feces with mild perianal staining; 3 points: severe diarrhea, indicated by watery feces and severe perianal staining.

Serum sample collection and determination: About 0.5 mL of blood was collected from the orbital venous plexus of surviving animals after isoflurane anesthesia. The blood was left to stand for 30-50 min at room temperature without anticoagulation, and then centrifuged at 3000 r/m for 15 min after serum was precipitated, and all the serum was separated out. The cytokines IL-4 and TNF-α were determined by using a liquid-phase chip assay kit.

Data statistics and analysis: Data for body weight, food intake, diarrhea scores, pathological examination results, among others, were expressed as mean ± standard deviation (Mean ± SD) and were statistically analyzed using SPSS statistical software 25.0.

### (2) Experimental results

1) Food intake: the food intake of animals in each group is shown in Table 7.

**Table 7. Statistical results of food intake of animals in each group (mean ± standard deviation)**

| Group | Daily average food intake (g) | | | | | |
|---|---|---|---|---|---|---|
| | Male | | | Female | | |
| | D4 | D8 | D12 | D4 | D8 | D12 |
| Blank group | 3.99±1.10 | 3.67±1.29 | 3.53±1.01 | 3.21±0.92 | 2.91±0.84 | 2.71±0.79 |
| Model group | 3.87±0.98 | 2.48±0.83 | 1.17±0.46 | 3.65±1.23 | 1.89±0.76 | 0.89±0.53 |
| Positive group 1 | 3.89±1.12 | 2.30±0.79 | 1.02±0.45 | 3.49±1.32 | 1.79±0.63 | 0.86±0.49 |
| Positive group 2 | 3.86±1.23 | 2.49±0.64 | 1.68±0.78 | 3.49±0.99 | 1.78±0.85 | 1.05±0.34 |
| NCTC 9343 inactivated bacteria powder low dose group | 3.98±0.87 | 2.35±0.79 | 1.35±0.32 | 3.67±0.77 | 1.87±0.63 | 0.92±0.28 |
| NCTC 9343 inactivated bacteria powder medium dose group | 4.12±0.79 | 2.39±0.88 | 1.43±0.68 | 3.54±0.98 | 1.86±0.74 | 0.89±0.76 |
| NCTC 9343 inactivated bacteria powder high dose group | 3.98±0.89 | 2.36±0.64 | 1.45±0.62 | 3.76±1.29 | 1.91±0.89 | 1.00±0.34 |
| ZY-312 inactivated bacteria powder low dose group | 4.02±1.32 | 2.49±0.53 | 1.68±0.71 | 3.41±1.13 | 2.01±0.92 | 1.17±0.87 |
| ZY-312 inactivated bacteria powder medium dose group | 4.10±1.28 | 2.48±0.67 | 1.79±0.67 | 3.56±1.24 | 1.91±1.01 | 1.1±0.34 |
| ZY-312 inactivated bacteria powder high dose group | 3.99±1.16 | 2.55±0.82 | 1.87±0.79 | 3.30±1.21 | 2.16±1.13 | 1.05±0.73 |

Prior to model induction (D4), there was no significant difference in food intake among the mice in all the groups. After the model induction (D8), the food intake of the mice in each group was reduced compared with the blank group. By the end of the experiment (D11), the food intake of the mice in each group was significantly reduced compared with the blank group. On D8, the food intake of the model group, the positive drug groups, and the NCTC 9343 low, medium, and high dose groups was similar, and the food intake of the ZY-312 low, medium, and high dose groups was higher than that of the groups described above; on D11, the food intake of the mice in the *Bacteroides fragilis* groups increased, with the increase of each ZY-312 group being greater than that of each NCTC 9343 group. There was no significant dose difference within the groups of each *Bacteroides fragilis* strain.
2) Moisture content of feces and diarrhea score: The statistical results of the moisture content of the feces of animals in each group are shown in Table 8.

**Table 8. Detection results of moisture content of feces of animals in each group (mean ± standard deviation, n = 10)**

| Group | Wet weight (g) | | Moisture content (%) | |
|---|---|---|---|---|
| | End of model induction | End of experiment | End of model induction | End of experiment |
| Blank group | 1.512±0.065 | 1.271±0.163** | 53.6±3.0** | 47.8±5.3** |
| Model group | 0.519±0.109 | 0.348±0.098 | 73.7±6.2 | 71.3±3.2 |
| Positive group 1 | 0.400±0.088 | 0.452±0.116 | 77.8±3.4 | 61.1±1.8 |
| Positive group 2 | 0.408±0.138 | 0.415±0.202 | 71.8±3.2 | 61.2±6.3 |
| NCTC 9343 inactivated bacteria powder low dose group | 0.397±0.056 | 0.422±0.093 | 77.4±5.4 | 62.5±5.2 |
| NCTC 9343 inactivated bacteria powder medium dose group | 0.413±0.083 | 0.416±0.129 | 78.5±4.9 | 62.8±3.9 |
| NCTC 9343 inactivated bacteria powder high dose group | 0.405±0.122 | 0.439±0.076 | 75.1±2.8 | 65.3±5.1 |
| ZY-312 inactivated bacteria powder low dose group | 0.411±0.057 | 0.473±0.122 | 76.7±2.1 | 56.8±3.8 |
| ZY-312 inactivated bacteria powder medium dose group | 0.358±0.063 | 0.455±0.125 | 72.6±2.1 | 57.5±2.4 |
| ZY-312 inactivated bacteria powder high dose group | 0.425±0.083 | 0.495±0.235 | 69.9±3.1 | 61.9±6.2 |

| | | | | |
|---|---|---|---|---|
| Note: * indicates significant difference p < 0.05 and ** indicates extremely significant difference p < 0.01, compared with the model group. | | | | |

The wet weight and moisture content of feces of mice in the blank group showed no significant difference throughout the experiment. After the model induction, the wet weight of the feces of mice in each group were significantly lower than that of the mice in the blank group, and the moisture content of the feces was significantly higher than that of the mice in the blank group. After the model induction, the wet weight and the moisture content of feces of the mice in the model group and in each treatment group were similar. By the end of the experiment, the wet weight of feces of mice in the model group naturally recovered, but there was no significant change in the moisture content of feces; positive group 1 and positive group 2 showed an increase in the wet weight of feces. Compared with the positive drug groups, in the *Bacteroides fragilis* groups, the wet weight of feces increased and the moisture content of feces was reduced, with the ZY-312 groups showing superior effects compared to the NCTC9343 groups; there was no significant dose dependence within the groups of each *Bacteroides fragilis* strain.

Diarrhea scores: the diarrhea scores of animals in each group are shown in Table 9.

**Table 9. Diarrhea scores of animals in each group (mean ± standard deviation, n = 5)**

| Group | Diarrhea determination time (day) | | | | |
|---|---|---|---|---|---|
| | 8 | 9 | 10 | 11 | 12 |
| Male | | | | | |
| Blank group | 0.0±0.0** | 0.0±0.0** | 0.0±0.0** | 0.0±0.0** | 0.0±0.0** |
| Model group | 1.9±0.2 | 2.0±0.0 | 2.8±0.4 | 3.0±0.0 | 2.3±0.7 |
| Positive group 1 | 1.8±0.4 | 2.3±0.5** | 2.7±0.3 | 2.7±0.3 | 1.8±0.6 |
| Positive group 2 | 2.0±0.0 | 2.4±0.7* | 2.7±0.3 | 2.8±0.5 | 1.7±0.7* |
| NCTC 9343 inactivated bacteria powder low dose group | 1.8±0.5 | 2.4±0.8 | 2.8±0.5 | 2.7±0.6 | 1.8±0.5 |
| NCTC 9343 inactivated bacteria powder medium dose group | 1.8±0.6 | 2.5±0.3 | 2.9±0.5 | 2.8±0.6 | 1.9±0.7 |
| NCTC 9343 inactivated bacteria powder high dose group | 1.7±0.3 | 2.5±0.4 | 3.0±0.0 | 2.8±0.3 | 1.8±0.6 |
| ZY-312 inactivated bacteria powder low dose group | 1.8±0.4 | 2.1±0.9 | 3.0±0.0 | 2.6±0.0 | 1.6±0.6 |
| ZY-312 inactivated bacteria powder medium dose group | 1.8±0.4 | 2.1±0.9 | 2.8±0.4 | 2.4±0.4 | 1.4±0.5** |
| ZY-312 inactivated bacteria powder high dose group | 1.7±0.7 | 2.0±1.2 | 2.7±0.7 | 2.3±0.5** | 1.3±0.5** |
| Female | | | | | |
| Blank group | 0.0±0.0** | 0.0±0.0** | 0.0±0.0** | 0.0±0.0** | 0.0±0.0** |
| Model group | 1.9±0.3 | 2.0±0.0 | 2.6±0.5 | 2.8±0.4 | 2.5±0.5 |
| Positive group 1 | 1.7±0.5 | 2.4±1.0 | 2.6±1.0 | 2.9±0.3 | 2.1±0.5 |
| Positive group 2 | 1.9±0.3 | 2.0±0.8 | 2.6±1.2 | 2.9±0.3 | 2.2±0.8 |
| NCTC 9343 inactivated bacteria powder low dose group | 1.8±0.4 | 2.4±0.7 | 2.5±1.1 | 2.8±0.5 | 2.2±0.5 |
| NCTC 9343 inactivated bacteria powder medium dose group | 1.9±0.3 | 2.3±0.9 | 2.6±0.9 | 2.8±0.7 | 2.1±0.2 |
| NCTC 9343 inactivated bacteria powder high dose group | 1.8±0.6 | 2.4±0.6 | 2.7±1.2 | 2.9±0.7 | 2.1±0.3 |
| ZY-312 inactivated bacteria powder low dose group | 1.7±0.5 | 1.3±1.2 | 2.2±1.0 | 2.0±0.5 | 1.3±0.9 |
| ZY-312 inactivated bacteria powder medium dose group | 1.7±0.5 | 1.2±1.0* | 2.2±1.2 | 2.0±1.0 | 1.4±0.8 |
| ZY-312 inactivated bacteria powder high dose group | 1.4±0.8 | 1.5±1.1 | 2.3±0.5 | 1.9±0.4** | 1.3±0.7* |

| | | | | | |
|---|---|---|---|---|---|
| Note: * indicates significant difference p < 0.05 and ** indicates extremely significant difference p < 0.01, compared with the model group. | | | | | |

For male animals, animals in the model group and each treatment group began to have diarrhea on day 4 after the model induction (D8), and the diarrhea scores were increased with the experiment time. Compared with the blank group, the diarrhea scores of the animals in the model group and each subject group after the model induction (D8-12) significantly increased (P < 0.01 or 0.05), indicating that the model induction was successful. The diarrhea score naturally declined on day 12 for the model group.

For male animals, the diarrhea score was reduced both in the positive group 1 and positive group 2. The *Bacteroides fragilis* groups exhibited the similar trend, but the ZY-312 groups down-regulated diarrhea scores more rapidly, and the degree was greater than that of the NCTC 9343 groups. There was no significant dose dependence within the groups of each *Bacteroides fragilis* strain. For female animals, the effect of each treatment group was similar to that for male animals.

3) Serum inflammatory cytokines in serum: the scores of serum inflammatory cytokines of animals in each group are shown in Table 10.

**Table 10. Scores of serum inflammatory cytokines of animals in each group (mean ± standard deviation, n = 10)**

| Group | TNF-α | IL-1β | IL-4 | IL-5 | IL-6 |
|---|---|---|---|---|---|
| Blank group | 0.17±0.02** | 7.64±0.33 | 2.14±1.98** | 0.64±0.18* | 1.08±0.00** |
| Model group | 0.51±0.12 | 8.29±1.21 | 4.46±2.35 | 2.09±1.96 | 8.45±6.75 |
| Positive group 1 | 0.67±0.40 | 8.13±0.80 | 3.70±1.17 | 0.79±0.50 | 19.21±19.20 |
| Positive group 2 | 0.46±0.22 | 7.64±0.33 | 3.03±1.40 | 1.80±0.93 | 14.32±6.95 |
| NCTC 9343 inactivated bacteria powder low dose group | 0.52±0.34 | 8.09±1.21 | 3.96±1.54 | 1.52±0.89 | 8.39±5.29 |
| NCTC 9343 inactivated bacteria powder medium dose group | 0.56±0.24 | 7.93±1.31 | 4.01±1.43 | 1.43±0.72 | 8.28±4.38 |
| NCTC 9343 inactivated bacteria powder high dose group | 0.57±0.37 | 8.01±1.24 | 4.03±1.36 | 1.56±0.83 | 8.76±4.79 |
| ZY-312 inactivated bacteria powder low dose group | 0.40±0.13* | 7.76±0.30 | 4.27±1.84 | 1.19±0.67* | 7.14±7.35 |
| ZY-312 inactivated bacteria powder medium dose group | 0.42±0.16 | 7.69±0.43 | 4.37±1.09 | 1.10±0.84 | 6.97±15.76 |
| ZY-312 inactivated bacteria powder high dose group | 0.37±0.31* | 7.61±0.32 | 4.48±0.97 | 1.09±1.33 | 6.85±6.75 |
| continued | | | | | |
| Group | IL-10 | IL-13 | IL-17 | IFN-γ | ICAM-1 |
| Blank group | 0.82±0.00 | 1.67±0.26 | 3.05±4.07 | 0.38±0.07 | 2687±830 |
| Model group | 0.99±0.34 | 2.07±1.18 | 1.79±0.44 | 0.78±0.23 | 3452±1582 |
| Positive group 1 | 1.70±1.31 | 1.94±0.27 | 1.55±6.21 | 0.80±1.88 | 2947±1430 |
| Positive group 2 | 0.82±0.82 | 1.90±0.55 | 1.69±0.23 | 0.47±0.10 | 2928±662 |
| NCTC 9343 inactivated bacteria powder low dose group | 1.26±0.45 | 1.92±0.34 | 1.66±0.69 | 0.56±0.47 | 2955±1239 |
| NCTC 9343 inactivated bacteria powder medium dose group | 1.33±0.42 | 1.88±0.43 | 1.73±0.73 | 0.62±0.87 | 2837±432 |
| NCTC 9343 inactivated bacteria powder high dose group | 1.29±0.72 | 1.81±0.52 | 1.69±0.89 | 0.59±0.39 | 2860±1988 |
| ZY-312 inactivated bacteria powder low dose group | 1.50±0.37 | 2.31±0.29 | 1.37±0.72 | 0.46±0.16 | 2629±1085 |
| ZY-312 inactivated bacteria powder medium dose group | 1.45±0.55 | 2.28±0.23 | 1.34±1.83 | 0.47±0.22 | 2528±1367 |
| ZY-312 inactivated bacteria powder high dose group | 1.46±0.87 | 2.30±0.55 | 1.28±1.24 | 0.45±0.31 | 2561±1835 |

| | | | | | |
|---|---|---|---|---|---|
| Note: * indicates significant difference p < 0.05 and ** indicates extremely significant difference p < 0.01, compared with the model group. | | | | | |

Compared with the blank group, the inflammatory cytokines (TNF-α, IL-1β, IL-5, IL-6, IL-17 and INF-γ) and ICAM-1 protein in the model group were significantly increased; while the anti-inflammatory cytokines IL-4, IL-10, and IL-13 were significantly reduced. This indicated that the positive drug groups effectively down-regulated the level of inflammatory cytokines and up-regulated the level of the anti-inflammatory cytokines. The *Bacteroides fragilis* groups also demonstrated cytokine regulation, with ZY-312 exhibiting a more effective regulation than NCTC 9343. There was no significant dose difference within the groups of each *Bacteroides fragilis* strain.

5) Pathological examination: Jejunum, ileum, and colorectum tissues were selected and HE staining was performed, followed by pathological damage evaluation.

**Table 11. Scores of degree of pathological damage in the intestinal tract of animals in each group (mean ± standard deviation, n = 10)**

| Group | Jejunum | | | |
|---|---|---|---|---|
| | Gland dilation | Intestinal mucosal damage | Inflammatory cell infiltration | Mucosal hyperemia |
| Blank group | 0±0** | 0±0 | 1.4±0.68 | 0.15±0.37* |
| Model group | 0.9±0.64 | 0.2±0.23 | 1.85±0.93 | 0.95±0.73 |
| Positive group 1 | 1.0±0.64 | 0.05±0.41 | 1.55±1.15 | 0.51±1.12 |
| Positive group 2 | 1.01±0.32 | 0.05±0.23 | 1.63±0.76 | 0.41±0.46* |
| NCTC 9343 inactivated bacteria powder low dose group | 0.95±0.52 | 0±0 | 1.66±0.56 | 0.72±0.36 |
| NCTC 9343 inactivated bacteria powder medium dose group | 0.9±0.64 | 0±0 | 3 67±0.47 | 0.7±0.86 |
| NCTC 9343 inactivated bacteria powder high dose group | 0.95±0.23 | 0±0 | 1.59±0.32 | 0.69±0.64 |
| ZY-312 inactivated bacteria powder low dose group | 0.72±0.34 | 0±0 | 1.49±0.76 | 0.53±0.7 |
| ZY-312 inactivated bacteria powder medium dose group | 0.7±0.23 | 0±0 | 1.48±0.51 | 0.42±0.61 |
| ZY-312 inactivated bacteria powder high dose group | 0.73±0.37 | 0±0 | 1.45±0.71 | 0.47±0.61 |

| Group | Ileum | | | |
|---|---|---|---|---|
| | Gland dilation | Intestinal mucosal damage | Inflammatory cell infiltration | Mucosal hyperemia |
| Blank group | 0±0** | 0±0* | 1.05±0.69 | 0.25±0.44 |
| Model group | 0.95±0.60 | 0.2±0.41 | 1.5±0.95 | 0.55±0.76 |
| Positive group 1 | 0.75±0.83 | 0.15±0.37 | 1.35±0.67 | 0.31±0.83 |
| Positive group 2 | 0.86±0.37 | 0.16±0.37 | 1.21±0.54 | 0.11±0.32 |
| NCTC 9343 inactivated bacteria powder low dose | 0.91±0.47 | 0.16±0.37 | 1.22±0.83 | 0.34±0.86 |
| NCTC 9343 inactivated bacteria powder medium dose | 0.85±0.23 | 0.13±0.46 | 1.26±0.68 | 0.37±0.23 |
| NCTC 9343 inactivated bacteria powder high dose | 0.75±0.60 | 0.15±0.32 | 1.19±0.46 | 0.30±0.41 |
| ZY-312 inactivated bacteria powder low dose group | 0.32±0.45 | 0.08±0.28 | 1.11±0.54 | 0.21±0.54 |
| ZY-312 inactivated bacteria powder medium dose group | 0.43±0.63 | 0.07±0.32 | 1.12±0.67 | 0.23±0.11 |
| ZY-312 inactivated bacteria powder high dose group | 0.29±0.56 | 0.09±0.32 | 1.05±0.67 | 0.21±0.42 |
| | | | | |

| Group | Colon | | | |
|---|---|---|---|---|
| | Gland dilation | Intestinal mucosal damage | | Mucosal hyperemia |
| Blank group | 0±0** | 0±0** | | 0±0** |
| Model group | 0.65±0.67 | 0.11±0.32 | | 0.05±0.23 |
| Positive group 1 | 0.6±1.14 | 0.05±0.22 | | 0.03±0.22 |
| Positive group 2 | 0.74±1.24 | 0.05±0.23 | | 0.01±0.23 |
| NCTC 9343 inactivated bacteria powder low dose group | 0.95±1.32 | 0±0** | | 0.02±0** |
| NCTC 9343 inactivated bacteria powder medium dose group | 0.91±0.93 | 0±0** | 0.03±0** | |
| NCTC 9343 inactivated bacteria powder high dose group | 0.99±0.79 | 0±0** | 0.03±0** | |
| ZY-312 inactivated bacteria powder low dose group | 0.34±0.33* | 0±0** | 0±0** | |
| ZY-312 inactivated bacteria powder medium dose group | 0.39±0.66 | 0±0** | 0±0** | |
| ZY-312 inactivated bacteria powder high dose group | 0.35±0.67 | 0±0** | 0±0** | |

| Group | Rectum | | | |
|---|---|---|---|---|
| | Gland dilation | Intestinal mucosal damage | Mucosal hyperemia | |
| Blank group | 0±0** | 0±0** | 0±0** | |
| Model group | 1.65±0.67 | 0.5±0.51 | 0.05±0.22 | |
| Positive group 1 | 1.30±0.98 | 0.5±0.51 | 0.35±0.59 | |
| Positive group 2 | 1.25±0.78 | 0.26±0.45 | 0.05±0.23 | |
| NCTC 9343 inactivated bacteria powder low dose group | 1.35±0.68 | 0.21±0.42 | 0.15±0.23 | |
| NCTC 9343 inactivated bacteria powder medium dose group | 1.39±0.77 | 0.26±0.45 | 0.13±0.23 | |
| NCTC 9343 inactivated bacteria powder high dose group | 1.36±0.82 | 0.23±0.32 | 0.12±0.34 | |
| ZY-312 inactivated bacteria powder low dose group | 0.95±0.67 | 0.12±0.47 | 0.05±0.22 | |
| ZY-312 inactivated bacteria powder medium dose group | 0.81±0.73 | 0.16±0.37 | 0.03±0.19 | |
| ZY-312 inactivated bacteria powder high dose group | 0.73±0.68 | 0.15±0.53 | 0±0** | |

For each intestinal tract section, compared with the blank group, pathological damages such as intestinal mucosal damage, significant gland dilation, inflammatory cell infiltration, mucosal hyperemia, and the like were found in the model group, and the positive group 1 and the positive group 2 showed therapeutic effect. The *Bacteroides fragilis* groups showed therapeutic effect, with the ZY-312 groups achieving superior therapeutic outcomes compared to the NCTC 9343 groups. There was no significant dose difference within the groups of each *Bacteroides fragilis* strain.

The *Bacteroides fragilis* inactivated bacteria powder (ZY-312/NCTC 9343) can treat the cancer treatment-induced diarrhea of mice caused by 5-FU, and the therapeutic effect of ZY-312 is better than that of NCTC 9343.

### Example 6. Therapeutic Effect of Bacteroides Fragilis Inactivated Bacteria Powder on Rat Models of Cancer Treatment-Induced Diarrhea Caused by Afatinib

### (1) Experimental method

Experimental design: 90 female SD rats were selected and randomly divided into 10 groups based on the body weight range. These included blank group, model group, positive group (Imodium 1.5 mg/kg), and NCTC 9343/ZY-312 inactivated bacteria powder low dose groups (10⁶ bacteria/rat), medium dose groups (10⁸ bacteria/rat), and high dose groups (10¹⁰ bacteria/rat), with 10 rats in each group. Except for the blank group, animals in the remaining groups were intragastrically given afatinib at a dose of 20 mg/kg (0.01 mL/10 g of body weight) once daily for 14 consecutive days to induce a diarrhea model. Starting from 3 days before model induction, animals in corresponding groups were intragastrically given (0.1 mL/10 g of body weight) Imodium suspension and NCTC 9343/ZY-312 bacteria powder suspensions at different concentrations (high, medium, and low doses), respectively, twice daily (once in the morning and once in the afternoon) for 14 consecutive days, while animals in the blank group and the model group received an equivalent volume of normal saline; the animals were euthanized after the final administration. General observation of animals and recording were carried out every day, the body weight was also recorded, and the diarrhea condition of each animal, as the focus, was observed and then recorded and scored. At the end of the experiment, all the surviving animals were euthanized and the entire intestinal tracts were harvested, fixed in 10% formaldehyde solution, and subjected to HE staining for pathological examination.

### Detection method and frequency:

Body weight: the body weight of animals was weighed daily using an electronic balance and recorded.

Diarrhea recording and scoring criteria: The diarrhea condition of each experimental rat was recorded daily, and the diarrhea scoring criteria are as follows:

| | |
|---|---|
| Normal | 0 points |
| Soft feces | 1 point |
| Shapeless feces and perianal skin and hair contamination | 2 points |
| Watery feces and perianal and hind leg contamination | 3 points |

Histopathological examination of intestinal tracts: All the surviving animals were euthanized and then the entire intestinal tracts were taken and fixed with 10% formaldehyde solution. Pathological tissue sections were prepared, and staining with HE was carried out; the histopathological examination of intestinal tracts was carried out under a microscope.

Data statistics and analysis: The data for body weight, food intake, diarrhea scores, pathological examination results, among others, were expressed as mean ± standard deviation (Mean ± SD) and were statistically analyzed using SPSS statistical software 25.0.

### (2) Experimental results

1) Body weight: the body weight of the animals in each group is shown in Table 12.

**Table 12. Body weight of animals in each group (mean ± standard deviation, n = 10)**

| Group | Body weight (g) | | | | |
|---|---|---|---|---|---|
| | D0 | D3 | D6 | D9 | D13 |
| Blank group | 216.2±7.5 | 225.4±9.4 | 231.7±9.4 | 240.6±11.1 | 253.7±10.3* |
| Model group | 215.3±8.0 | 223.7±11.7 | 212.7±10.6 | 202.3±16.1 | 206.0±21.0 |
| Positive group | 214.9±8.7 | 225.8±8.3 | 225.3±8.7 | 217.3±12.4 | 219.5±21.49 |
| NCTC 9343 inactivated bacteria powder low dose group | 215.0±6.3 | 223.9±16.7 | 217.6±12.6 | 208.8±13.1 | 213.0±13.4 |
| NCTC 9343 inactivated bacteria powder medium dose group | 211.4±9.2 | 227±14.5 | 220.8±13.0 | 212.1±6.1 | 210.6±16.5 |
| NCTC 9343 inactivated bacteria powder high dose group | 212.6±10.8 | 227.6±18.2 | 222.0±14.2 | 217.4±9.1 | 219.1±13.0 |
| ZY-312 inactivated bacteria powder low dose group | 214.5±8.1 | 224.9±8.9 | 223.2±12.2 | 223.0±15.2 | 225.1±9.8 |
| ZY-312 inactivated bacteria powder medium dose group | 216.0±9.0 | 225±10.3 | 225.9±11.4 | 223.9±13.6 | 227.2±14.8 |
| ZY-312 inactivated bacteria powder high dose group | 215.9±6.2 | 223.8±9.8 | 221.1±14.8 | 222.7±15.2 | 221.2±12.7 |

| | | | | | |
|---|---|---|---|---|---|
| Note: * indicates significant difference p < 0.05 and ** indicates extremely significant difference p < 0.01, compared with the model group. | | | | | |

Before the start of the experiment (D0) and prior to model induction (D3), the body weights of the animals across all groups were comparable, with the animals in the blank group, the model group, and each treatment group experiencing similar rates of weight gain. Starting from D3, the body weight of the animals in the model group was in a descending trend, with the body weight changes in treatment group mirroring that of the model group. Specially, the average body weight of the model group was maintained at 220 g, dropping to 210 g by D13; the average body weight of the positive group was similar to that of the model group and was greatly reduced on D13 to below 210 g. The average body weight of each NCTC 9343 treatment group started to decline from D6, stabilizing around 210 g from D9 to D13; meanwhile, the average body weight of each ZY-312 group did not change much, fluctuating around 220 g from D6 to D13. In terms of body weight, there was no significant difference between the treatment groups.
2) Diarrhea scores: the diarrhea scores of animals in each group are shown in Table 13.

**Table 13. Diarrhea scores of animals in each group (mean ± standard deviation, n = 10)**

| Group | Diarrhea score | | | | |
|---|---|---|---|---|---|
| | D4 | D6 | D9 | D11 | D13 |
| Blank group | 0±0* | 0±0** | 0±0** | 0±0** | 0±0** |
| Model group | 0.8±0.6 | 1.7±0.5 | 1.9±0.3 | 2.1±0.0 | 2.6±0.2 |
| Positive group | 0.8±0.6 | 1.4±0.4 | 1.6±0.3 | 1.8±0.0 | 2.1±0.2 |
| NCTC 9343 bacteria powder low dose group | 0.8±0.8 | 1.3±0.5 | 1.7±0.2 | 2.0±0.5 | 2.4±0.5 |
| NCTC 9343 bacteria powder medium dose group | 0.9±0.9 | 1.5±0.5 | 1.6±0.2 | 1.8±0.4 | 2.1±0.5 |
| NCTC 9343 bacteria powder high dose group | 1.0±0.7 | 1.5±0.5 | 1.7±0.5 | 1.8±0.4 | 2.3±0.4 |
| ZY-312 bacteria powder low dose group | 0.8±0.4 | 1.4±0.5 | 1.4±0.2 | 1.6±0.2 | 2.1±0.5 |
| ZY-312 bacteria powder medium dose group | 0.7±0.7 | 1.3±0.5 | 1.4±0.5 | 1.5±0.2 | 2.0±0.5 |
| ZY-312 bacteria powder high dose group | 0.6±0.5 | 1.4±0.5 | 1.5±0.5 | 1.6±0.2 | 2.0±0.6* |

| | | | | | |
|---|---|---|---|---|---|
| Note: * indicates significant difference p < 0.05 and ** indicates extremely significant difference p < 0.01, compared with the model group. | | | | | |

The animals in each group began to exhibit diarrhea the next day after modeling (D4). Compared with the blank group, the diarrhea scores of the model group and each treatment group during the experiment were significantly increased, and escalated over time. The positive group and the NCTC 9343 groups demonstrated a similar upward trend in diarrhea scores to that of the model group. In contrast, ZY-312 showed the ability to maintain a stable diarrhea score. Diarrhea scores on D6 were slightly less than those of the other groups; the suppressive trend of the low dose and the medium dose remained to D13, and the diarrhea score at a high dose was significantly lower than the model group on D13; the suppressive trend of the medium dose was maintained to D11. Thus, all treatment groups exhibited the ability to suppress the rise in diarrhea score, wherein the effect of ZY-312 was better than the positive group and the NCTC 9343 groups.
3) Histopathological examination of intestinal tracts: jejunum, ileum, and colorectum tissues were selected to evaluate the degree of mucosal hyperemia and damage. The results are shown in Table 14.

**Table 14. Pathological examination results of animals in each group (mean ± standard deviation, n = 10)**

| Group | Jejunum | | Ileum | | Colorectum | |
|---|---|---|---|---|---|---|
| | Gland dilation | Mucosal hyperemia | Gland dilation | Mucosal hyperemia | Gland dilation | Mucosal hyperemia |
| Blank group | 0.0±0.0 | 0.0±0.0 | 0.0 ± 0.0 | 0.03±0.24 | 0.0±0.0 | 0.0±0.0 |
| Model group | 0.75±0.67 | 0.57±0.59 | 2.55±1.56 | 1.09±1.17 | 0.26±0.24 | 0.47±0.36 |
| Positive group | 0.47±0.84 | 0.44±0.72 | 2.42±1.34 | 0.95±1.02 | 0.19±0.29 | 0.27±0.79 |
| NCTC 9343 inactivated bacteria powder low dose group | 0.44±0.38 | 0.48±0.41 | 1.68±0.61 | 0.73±0.65 | 0.16±0.37 | 0.23±0.40 |
| NCTC 9343 inactivated bacteria powder medium dose group | 0.48±0.32 | 0.45±0.77 | 1.48±0.82 | 0.72±0.68 | 0.12±0.58 | 0.25±0.48 |
| NCTC 9343 inactivated bacteria powder high dose group | 0.46±0.71 | 0.42±0.25 | 1.50±0.79 | 0.79±1.20 | 0.14±0.68 | 0.25±0.57 |
| ZY-312 inactivated bacteria powder low dose group | 0.22±0.47 | 0.38±0.69 | 0.99±0.71 | 0.68±1.19 | 0.12±0.23 | 0.15±0.46 |
| ZY-312 inactivated bacteria powder medium dose group | 0.23±0.56 | 0.27±0.39 | 1.06±0.87 | 0.65±0.77 | 0.07±0.31 | 0.09±0.67 |
| ZY-312 inactivated bacteria powder high dose group | 0.14±0.31 | 0.24±0.63 | 1.04±0.52 | 0.62±0.69 | 0.06±0.51 | 0.06±0.43 |

For the intestinal tract sections, compared with the blank group, the model group exhibited significant gland dilation and mucosal hyperemia, each treatment group showed therapeutic effect on the damages described above, NCTC 9343 exhibited a therapeutic effect comparable to the positive control, and the ZY-312 groups outperformed the NCTC 9343 groups in terms of therapeutic efficacy.

In conclusion, the *Bacteroides fragilis* inactivated bacteria powder (ZY-312/NCTC 9343) can treat weight loss, chemotherapy-related diarrhea, intestinal mucosal damage, mucosal hyperemia, inflammatory cell infiltration, and gland damage in rats caused by afatinib, with ZY-312 showing superior therapeutic effects compared to NCTC 9343.

Embodiments of the present disclosure have been described above. However, the present disclosure is not limited thereto. Any modification, equivalent replacement, improvement, and the like made without departing from the spirit and principle of the present disclosure shall all fall within the protection scope of the present disclosure.

## Claims

1. Use of *Bacteroides fragilis* in the preparation of a composition for preventing and/or treating cancer treatment-induced diarrhea.

2. The use according to claim 1, wherein the *Bacteroides fragilis* is selected from *Bacteroides fragilis* ZY-312 with the preservation number of CGMCC No. 10685.

3. Use of *Bacteroides fragilis* in the preparation of a composition for preventing and/or treating side effects of chemotherapeutic or targeted drugs.

4. The use according to claim 3, wherein the *Bacteroides fragilis* is selected from *Bacteroides fragilis* ZY-312 with the preservation number of CGMCC No. 10685.

5. The use according to claim 4, wherein the chemotherapeutic drug is selected from one or more of the group consisting of cytotoxic chemotherapeutic drugs, taxanes, platinum-based drugs, fluorouracils, and camptothecins; and/or, the targeted drug is selected from one or more of the group consisting of Anti-EGFR, Anti-HER-2, Anti-BRAF, Anti-MEK, Anti-EMI,4/ALK, Anti-VEGF, Multi-targeted TKI, Anti-mTOR, Anti-CDK4/6, and Anti-PARP;
preferably, the chemotherapeutic drug is selected from one or more of the group consisting of docetaxel, oxaliplatin, cisplatin, fluorouracil, capecitabine, and irinotecan; and/or, the targeted drug is selected from one or more of the group consisting of afatinib, osimertinib, neratinib, vemurafenib, dabrafenib, cobimetinib, trametinib, crizotinib, aflibercept, lapatinib, pyrotinib, imatinib, pazopanib, everolimus, sirolimus, palbociclib, ribociclib, olaparib, and rucaparib.

6. The use according to any one of claims 1-3, wherein the *Bacteroides fragilis* is one or more of the group consisting of: living *Bacteroides fragilis; Bacteroides fragilis* that has been subjected to the following treatments: inactivation, genetic recombination, engineering or modification, attenuation, chemical treatment, or physical treatment or inactivated *Bacteroides fragilis; Bacteroides fragilis* lysate; and *Bacteroidesfragilis* liquid culture supernatant.

7. The use according to claim 6, wherein the inactivated *Bacteroides fragilis* is a morphologically intact inactivated bacterium and/or a morphologically incomplete inactivated bacterium, preferably a morphologically intact inactivated bacterium.

8. The use according to claim 7, wherein the *Bacteroides fragilis* is inactivated by any one or more of the group consisting of the methods including dry heating, moist heating, filtration, organic solvents, chemical reagents, ultraviolet or infrared radiation, fermentation, lyophilization, genetic recombination, and genetic modification or engineering;
preferably, inactivated bacteria powders are obtained via lyophilization.

9. The use according to any one of claims 1-3, wherein the composition is any one of a pharmaceutical composition, food, a healthcare product, or a food additive.

10. The use according to claim 9, wherein the pharmaceutical composition comprises a pharmaceutically effective amount of the inactivated *Bacteroides fragilis* ZY-312 with the preservation number of CGMCC No. 10685; and/or, the pharmaceutical composition further comprises a pharmaceutically acceptable carrier and/or excipients; and/or, the pharmaceutical composition is administered orally or by enema.
